(19) Europäisches Patentamt
European Patent Office
Office européen des brevets

(11) Veröffentlichungsnummer: **0 355 540 A2**

(12) **EUROPÄISCHE PATENTANMELDUNG**

(21) Anmeldenummer: **89114517.9**

(22) Anmeldetag: **05.08.89**

(51) Int. Cl.4: **C07D 295/033 , C07D 211/14 , C07D 265/30 , C07D 307/14 , C07D 309/04 , C07D 317/00 , C07D 319/00 , A01N 33/04 , A01N 43/00**

(30) Priorität: **19.08.88 DE 3828194**

(43) Veröffentlichungstag der Anmeldung: **28.02.90 Patentblatt 90/09**

(84) Benannte Vertragsstaaten: **BE CH DE FR GB IT LI NL**

(71) Anmelder: **BAYER AG**
**D-5090 Leverkusen 1 Bayerwerk(DE)**

(72) Erfinder: **Weissmueller, Joachim, Dr.**
**Carl-Langhans-Strasse 53**
**D-4019 Monheim(DE)**
Erfinder: **Dutzmann, Stefan, Dr.**
**Leinenweberweg 33**
**D-4000 Duesseldorf 13(DE)**

(54) **Substituierte Cyclohexylamine, Verfahren zu deren Herstellung sowie deren Verwendung in Schädlingsbekämpfungsmitteln.**

(57) Substituierte Cyclohexylamine der allgemeinen Formel (I),

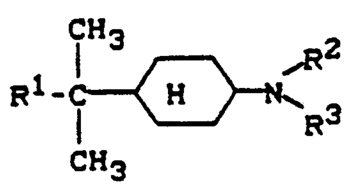

(I)

in welcher
$R^1$ für Wasserstoff, Alkyl, Cycloalkyl, Cycloalkylalkyl, Aralkyl oder Aryl steht,
$R^2$ für Tetrahydrofuranylalkyl, Tetrahydropyranylalkyl, Dioxolanylalkyl oder Dioxanylalkyl steht und
$R^3$ für Wasserstoff, Alkyl, für unsubstituiertes oder substituiertes Aralkyl, für Tetrahydrofuranylalkyl, Tetrahydropyranylalkyl, Dioxolanylalkyl oder Dioxanylalkyl steht oder
$R^2$ und $R^3$ gemeinsam mit dem Stickstoffatom, an welches sie gebunden sind, für einen unsubstituierten oder substituierten Heterocyclus stehen,
sowie deren Verwendung in Schädlingsbekämpfungsmitteln. Die neuen Cyclohexylamine können hergestellt werden, wenn man z.B. geeignete Aniline mit Wasserstoff in Gegenwart eines geeigneten Katalysators und gegebenenfalls in Gegenwart eines geeigneten Verdünnungsmittels hydriert.

EP 0 355 540 A2

Xerox Copy Centre

## Substituierte Cyclohexylamine, Verfahren zu deren Herstellung sowie deren Verwendung in Schädlingsbekämpfungsmitteln

Die Erfindung betrifft neue substituierte Cyclohexylamine, mehrere Verfahren zu ihrer Herstellung und ihre Verwendung in Schädlingsbekämpfungsmitteln.

Es ist bekannt, daß bestimmte Cycloalkylaminoverbindungen, wie beispielsweise die Verbindung 2-[2-Methyl-3-(3,5-dimethylpiperidin-1-yl)-propyl]-6-methyldecalin fungizide Eigenschaften besitzen (vgl. z.B. EP 254 150).

Die Wirksamkeit dieser vorbekannten Verbindungen ist jedoch insbesondere bei niedrigen Aufwandmengen und Konzentrationen nicht in allen Anwendungsgebieten völlig zufriedenstellend.

Es wurden neue substituierte Cyclohexylamine der allgemeinen Formel (I),

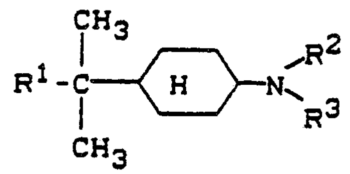

(I)

in welcher

$R^1$ für Wasserstoff, Alkyl, Cycloalkyl, Cycloalkylalkyl, Aralkyl oder Aryl steht,

$R^2$ für Tetrahydrofuranylalkyl, Tetrahydropyranylalkyl, Dioxolanylalkyl oder Dioxanylalkyl steht und

$R^3$ für Wasserstoff, Alkyl, für unsubstituiertes oder substituiertes Aralkyl, für Tetrahydrofuranylalkyl, Tetrahydropyranylalkyl, Dioxolanylalkyl oder Dioxanylalkyl steht oder

$R^2$ und $R^3$ gemeinsam mit dem Stickstoffatom, an welches sie gebunden sind, für einen unsubstituierten oder substituierten Heterocyclus stehen,

sowie deren Säureadditionssalze gefunden.

Die Verbindungen der Formel (I) können als geometrische und gegebenenfalls auch als optische Isomere oder Isomerengemische unterschiedlicher Zusammensetzung vorliegen. Sowohl die reinen Isomeren als auch die Isomerengemische werden erfindungsgemäß beansprucht.

Weiterhin wurde gefunden, daß man die neuen substituierten Cyclohexylamine der allgemeinen Formel (I),

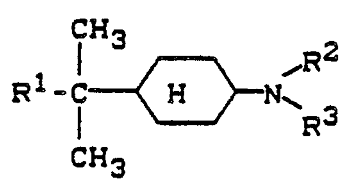

(I)

in welcher

$R^1$ für Wasserstoff, Alkyl, Cycloalkyl, Cycloalkylalkyl, Aralkyl oder Aryl steht,

$R^2$ für Tetrahydrofuranylalkyl, Tetrahydropyranylalkyl, Dioxolanylalkyl oder Dioxanylalkyl steht und

$R^3$ für Wasserstoff, Alkyl, für unsubstituiertes oder substituiertes Aralkyl, für Tetrahydrofuranylalkyl, Tetrahydropyranylalkyl, Dioxolanylalkyl oder Dioxanylalkyl steht oder

$R^2$ und $R^3$ gemeinsam mit dem Stickstoffatom, an welches sie gebunden sind, für einen unsubstituierten oder substituierten Heterocyclus stehen,

sowie deren Säureadditionssalze erhält, wenn man

(a) Cyclohexanone der Formel (II),

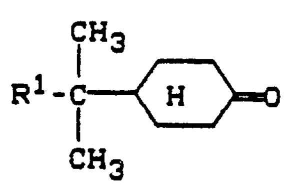

(II)

in welcher
$R^1$ die oben angegebene Bedeutung hat,
mit Aminen der Formel (III),

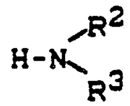

(III)

in welcher
$R^2$ und $R^3$ die soben angegebene Bedeutung haben,
in Gegenwart eines Reduktionsmittels, gegebenenfalls in Gegenwart eines Verdünnungsmittels und gegebenenfalls in Gegenwart eines Reaktionshilfsmittels umsetzt, oder wenn man

(b) substituierte Aniline der Formel (IV),

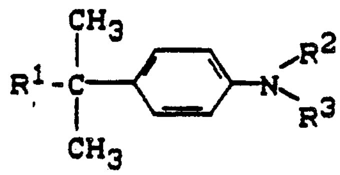

(IV)

in welcher
$R^1$, $R^2$ und $R^3$ die oben angegebene Bedeutung haben,
mit Wasserstoff in Gegenwart eines Katalysators und gegebenenfalls in Gegenwart eines Verdünnungsmittels hydriert;
desweiteren erhält man substituierte Cyclohexylamine der Formel (Ia),

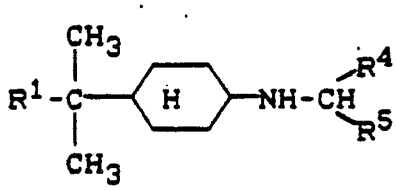

(Ia)

in welcher
$R^4$ für Wasserstoff oder Alkyl steht,
$R^5$ für Tetrahydrofuranyl, Tetrahydropyranyl, Dioxolanyl, Dioxanyl, Tetrahydrofuranylalkyl, Tetrahydropyranylalkyl, Dioxolanylalkyl oder Dioxanylalkyl steht und
$R^1$ die oben angegebene Bedeutung hat,
alternativ auch, wenn man

(c) Cyclohexylamine der Formel (V),

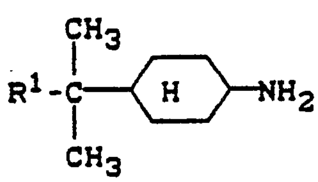

(V)

in welcher
$R^1$ die oben angegebene Bedeutung hat,
mit Aldehyden oder Ketonen der Formel (VI),

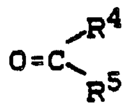

(VI)

in welcher
$R^4$ und $R^5$ die oben angegebene Bedeutung haben,
in Gegenwart eines Reduktionsmittels, gegebenenfalls in Gegenwart eines Verdünnungsmittels und gegebenenfalls in Gegenwart eines Reaktionshilfsmittels umsetzt;
ferner erhält man substituierte Cyclohexylamine der Formel (Ib),

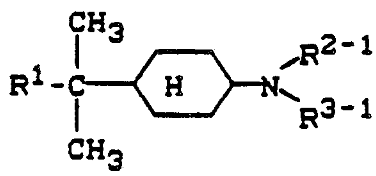

(Ib)

in welcher
$R^{2-1}$ für Tetrahydrofuranylalkyl, Tetrahydropyranylalkyl, Dioxolanylalkyl oder Dioxanylalkyl steht,
$R^{3-1}$ für Wasserstoff, Alkyl, für unsubstituiertes oder substituiertes Aralkyl, für Tetrahydrofuranylalkyl, Tetrahydropyranylalkyl, Dioxolanylalkyl oder Dioxanylalkyl steht und
$R^1$ die oben angegebene Bedeutung hat,
alternativ auch, wenn man

(d) Cyclohexylamine der Formel (VII),

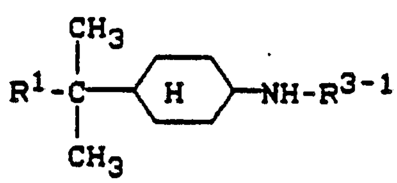

(VII)

in welcher
$R^1$ und $R^{3-1}$ die oben angegebene Bedeutung haben,
mit Alkylierungsmitteln der Formel (VIIIa),
$R^{2-1}$ - $E^1$ (VIIIa)
in welcher
$R^{2-1}$ die oben angegebene Bedeutung hat und
$E^1$ für eine elektronenanziehende Abgangsgruppe steht,
oder wenn man

(e) substituierte Cyclohexylamine der Formel (Ic),

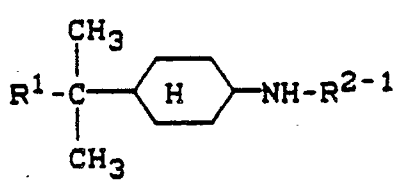

(Ic)

in welcher
$R^1$ und $R^{2-1}$ die oben angegebene Bedeutung haben,
mit Alkylierungsmitteln der Formel (VIIIb),
$R^{3-2}$ - $E^2$ (VIIIb)
in welcher
$R^{3-2}$ für Alkyl oder für unsubstituiertes oder substituiertes Aralkyl steht und

$E^2$ für eine elektronenanziehende Abgangsgruppe steht,
jeweils gegebenenfalls in Gegenwart eines Verdünnungsmittels und gegebenenfalls in Gegenwart eines Reaktionshilfsmittels umsetzt
und gegebenenfalls anschließend eine Säure addiert.

Schließlich wurde gefunden, daß die neuen substituierten Cyclohexylamine der allgemeinen Formel (I) und deren Säureadditionssalze ausgezeichnete Wirksamkeit gegen Schädlinge besitzen.

Überraschenderweise zeigen die erfindungsgemäßen substituierten Cyclohexylamine der allgemeinen Formel (I) zum Beispiel eine deutlich bessere fungizide Wirksamkeit als die aus dem Stand der Technik bekannten wirkungsmäßig naheliegenden Cycloalkylaminoverbindungen.

Die erfindungsgemäßen substituierten Cyclohexylamine sind durch die Formel (I) allgemein definiert.

Alkyl steht in weiteren, falls nicht anders definiert, in einzelnen oder zusammengesetzten Resten und Substituenten für geradkettiges oder verzweigtes Alkyl mit 1 bis 6 und vorzugsweise 1 bis 4 Kohlenstoffatomen. Beispielhaft genannt seien Methyl, Ethyl, n- und i-Propyl sowie n-, i-, s- und t-Butyl.

Cycloalkyl und Cycloalkylalkyl steht im weiteren, falls nicht anders definiert, für Cycloalkyl mit 3 bis 7, vorzugsweise 5 bis 6 Kohlenstoffatomen in Cycloalkylteil. Beispielhaft genannt seien Cyclopentyl, Cyclohexyl, Cyclohexylmethyl, Cyclohexylethyl und Cyclohexylpropyl.

Aryl bzw. Aralkyl steht im weiteren vorzugsweise für Phenyl, Naphthyl bzw. Phenylalkyl oder Naphthylalkyl. Beispielhaft genannt seien Phenyl, Benzyl, Phenylethyl und Phenylpropyl.

Der von $R^2$ und $R^3$ gemeinsam mit dem Stickstoffatom gebildete Heterocyclus ist zum Beispiel ein fünf- bis siebengliedriger Heterocyclus, der gegebenenfalls ein weiteres Heteroatom, insbesondere Stickstoff, Sauerstoff oder Schwefel enthalten kann. Bevorzugte Heterocyclen sind Pyrrolidin, Piperidin, Perhydroazepin und Morpholin.

Halogen steht im weiteren für Fluor, Chlor, Brom und Iod.

Bevorzugt sind die Verbindungen der Formel (I), in denen
$R^1$ für Wasserstoff, für geradkettiges oder verzweigtes Alkyl mit 1 bis 6 Kohlenstoffatomen, für Cycloalkyl mit 3 bis 7 Kohlenstoffatomen, für Cycloalkylalkyl mit 3 bis 7 Kohlenstoffatomen im Cycloalkylteil und 1 bis 6 Kohlenstoffatomen im geradkettigen oder verzweigten Alkylteil, für Phenylalkyl mit 1 bis 6 Kohlenstoffatomen im geradkettigen oder verzweigten Alkylteil oder für Phenyl oder Naphthyl steht,
$R^2$ für Tetrahydrofuranylalkyl, Tetrahydropyranylalkyl, Dioxolanylalkyl oder Dioxanylalkyl mit jeweils 1 bis 4 Kohlenstoffatomen im geradkettigen oder verzweigten Alkylteil steht und
$R^3$ für Wasserstoff, für geradkettiges oder verzweigtes Alkyl mit 1 bis 6 Kohlenstoffatomen oder für unsubstituiertes oder einfach oder mehrfach, gleich oder verschieden substituiertes Aralkyl mit 1 bis 6 Kohlenstoffatomen im geradkettigen oder verzweigten Alkylteil und 6 bis 10 Kohlenstoffatomen im Arylteil steht, wobei als Arylsubstituenten infrage kommen: Halogen, Cyano, Nitro, jeweils geradkettiges oder verzweigtes Alkyl, Alkoxy oder Alkylthio mit jeweils 1 bis 4 Kohlenstoffatomen, jeweils geradkettiges oder verzweigtes Halogenalkyl, Halogenalkoxy oder Halogenalkylthio mit jeweils 1 bis 4 Kohlenstoffatomen und 1 bis 9 gleichen oder verschiedenen Halogenatomen, jeweils geradkettiges oder verzweigtes Alkoxycarbonyl oder Alkoximinoalkyl mit jeweils 1 bis 4 Kohlenstoffatomen in den einzelnen Alkylteilen sowie unsubstituiertes oder einfach oder mehrfach, gleich oder verschieden durch Halogen und/oder geradkettiges oder verzweigtes Alkyl mit 1 bis 4 Kohlenstoffatomen substituiertes Phenyl; oder für Tetrahydrofuranylalkyl, Tetrahydropyranylalkyl, Dioxolanylalkyl oder Dioxanylalkyl mit jeweils 1 bis 4 Kohlenstoffatomen im geradkettigen oder verzweigten Alkylteil steht oder
$R^2$ und $R^3$ gemeinsam mit dem Stickstoffatom, an welches sie gebunden sind, für einen unsubstituierten oder einfach oder mehrfach, gleich oder verschieden substituierten gesättigten fünf- bis siebengliedrigen Heterocyclus stehen, der gegebenenfalls ein weiteres Heteroatom -insbesondere Stickstoff, Sauerstoff oder Schwefel - enthalten kann, wobei als Substituenten infrage kommen: jeweils geradkettiges oder verzweigtes Alkyl oder Hydroxyalkyl mit jeweils 1 bis 4 Kohlenstoffatomen.

Besonders bevorzugt sind Verbindungen der Formel (I), in denen
$R^1$ für Wasserstoff, Methyl, Ethyl, n- oder i-Propyl, Cyclopentyl, Cyclohexyl, Cyclohexylmethyl, Cyclohexylethyl, Cyclohexylpropyl, Phenyl, Benzyl, Phenylethyl oder Phenylpropyl steht,
$R^2$ für Tetrahydrofuranylmethyl, Tetrahydropyranylmethyl, Dioxolanylmethyl oder Dioxanylmethyl steht und
$R^3$ für Wasserstoff, Methyl, Ethyl, n- oder i-Propyl, n-, i-, s- oder t-Butyl oder für unsubstituiertes oder ein- bis dreifach, gleich oder verschieden substituiertes Phenylalkyl mit 1 bis 4 Kohlenstoffatomen im geradkettigen oder verzweigten Alkylteil steht, wobei als Phenylsubstituenten infrage kommen:
Fluor, Chlor, Brom, Cyano, Nitro, Methyl, Ethyl, n- oder i-Propyl, n-, i-, s- oder t-Butyl, Methoxy, Ethoxy, n- oder i-Propoxy, Methylthio, Trifluormethyl, Trifluormethoxy, Trifluormethylthio oder unsubstituiertes oder ein- bis dreifach, gleich oder verschieden durch Fluor, Chlor, Brom, Methyl und/oder Ethyl substituiertes Phenyl; oder für Tetrahydrofuranylmethyl, Tetrahydropyranylmethyl, Dioxolanylmethyl, Dioxanylmethyl steht oder

$R^2$ und $R^3$ gemeinsam mit dem Stickstoffatom, an welches sie gebunden sind, für einen unsubstituierten oder ein- bis dreifach, gleich oder verschieden durch Methyl, Ethyl und/oder Hydroxymethyl substituierten 1-Pyrrolidinylrest, 1-Piperidinylrest, 1-Perhydroazepinylrest oder 4-Morpholinylrest stehen.

Bevorzugte erfindungsgemäße Verbindungen sind auch Additionsprodukte, insbesondere die pflanzenverträglichen Additionsprodukte, aus Säuren und denjenigen substituierten Cyclohexylaminen der Formel (I), in denen die Substituenten $R^1$, $R^2$ und $R^3$ die Bedeutungen haben, die bereits vorzugsweise für diese Substituenten genannt wurden.

Zu den Säuren die addiert werden können, gehören vorzugsweise Halogenwasserstoffsäuren, wie. z.B. Chlorwasserstoffsäure und Bromwasserstoffsäure, insbesondere Chlorwasserstoffsäure, ferner Phosphorsäure, Salpetersäure, Schwefelsäure, mono-, bi- und trifunktionelle Carbonsäuren und Hydroxycarbonsäuren, wie. z.B. Essigsäure, Maleinsäure, Bernsteinsäure, Fumarsäure, Weinsäure, Zitronensäure, Salicylsäure, Sorbinsäure und Milchsäure, Sulfonsäuren, wie z.B. p-Toluolsulfonsäure und 1,5-Naphthalindisulfonsäure sowie Saccharin oder Thiosaccharin.

Im einzelnen seien außer den bei den Herstellungsbeispielen genannten Verbindungen die folgenden substituierten Cyclohexylamine der allgemeinen Formel (I) genannt:

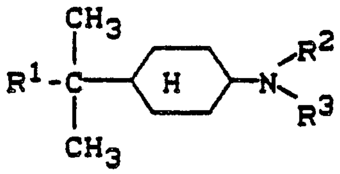 (I)

| $R^1$ | 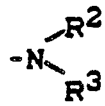 | $R^1$ | 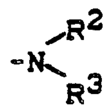 |
|---|---|---|---|
| $(CH_3)_2CH-$ | 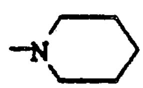 | H | 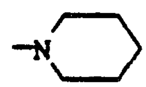 |
| $(CH_3)_2CH-$ | 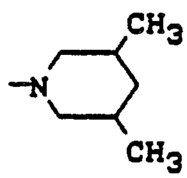 | H | 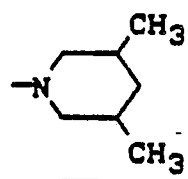 |
| $(CH_2)_2CH-$ | 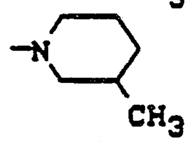 | H | 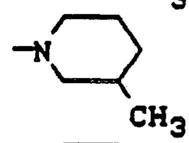 |
| $(CH_3)_2CH-$ | 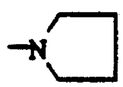 | H | 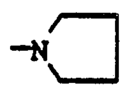 |

| $R^1$ | 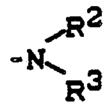 | $R^1$ | 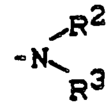 |
|---|---|---|---|
| $(CH_3)_2CH-$ | 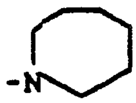 | H | 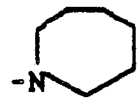 |
| $(CH_3)_2CH-$ | 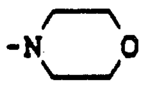 | H | 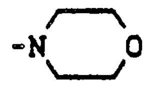 |
| $(CH_3)_2CH-$ | 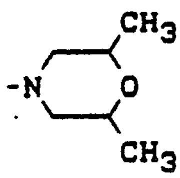 | H | 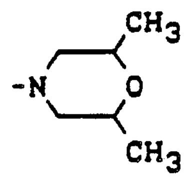 |
| $(CH_3)_2CH-$ | 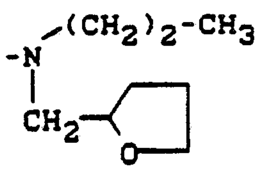 | H | 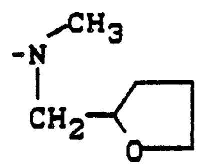 |
| $(CH_3)_2CH-$ | 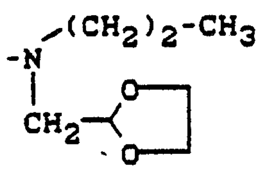 | $CH_3$ | 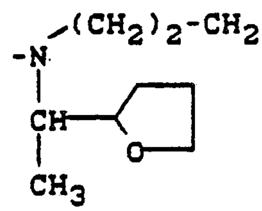 |
| $(CH_3)_2CH-$ | 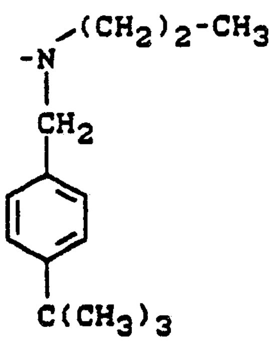 | $CH_3$ | 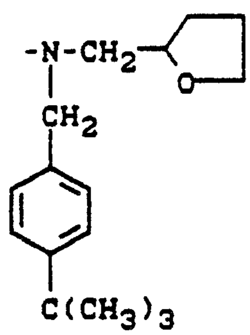 |

| $R^1$ | 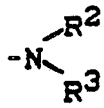 | $R^1$ | 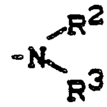 |
|---|---|---|---|
| $C_2H_5$ | 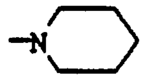 | $CH_3(CH_2)_2$- | 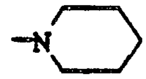 |
| $C_2H_5$ | 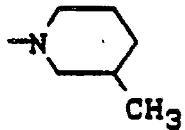 | $CH_3(CH_2)_2$- | 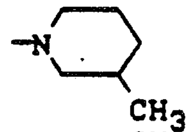 |
| $C_2H_5$ | 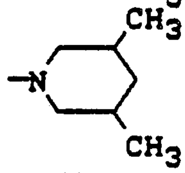 | $CH_3(CH_2)_2$- | 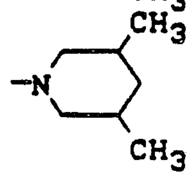 |
| $C_2H_5$ | 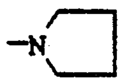 | $CH_3(CH_2)_2$- | 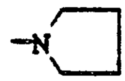 |
| $C_2H_5$ | 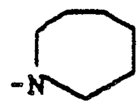 | $CH_3(CH_2)_2$- | 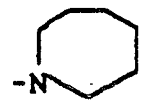 |
| $C_2H_5$ | 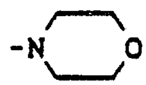 | $CH_3(CH_2)_2$- | 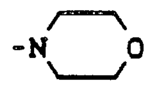 |
| $C_2H_5$ | 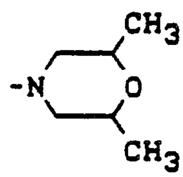 | $CH_3(CH_2)_2$- | 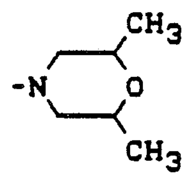 |
| $C_2H_5$ | 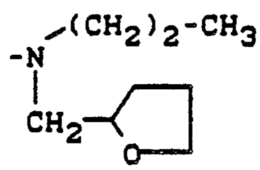 | $CH_3(CH_2)_2$- | 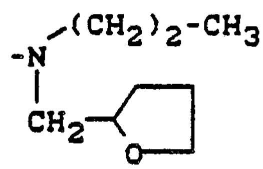 |
| $C_2H_5$ | 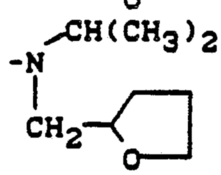 | $CH_3(CH_2)_2$- | 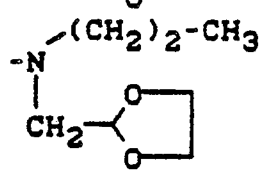 |
| $C_2H_5$ | 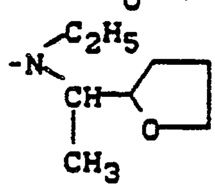 | $CH_3(CH_2)_2$- | 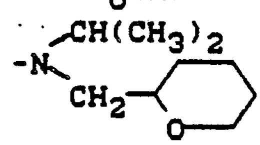 |
| | | $CH_3(CH_2)_2$- | 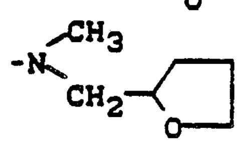 |

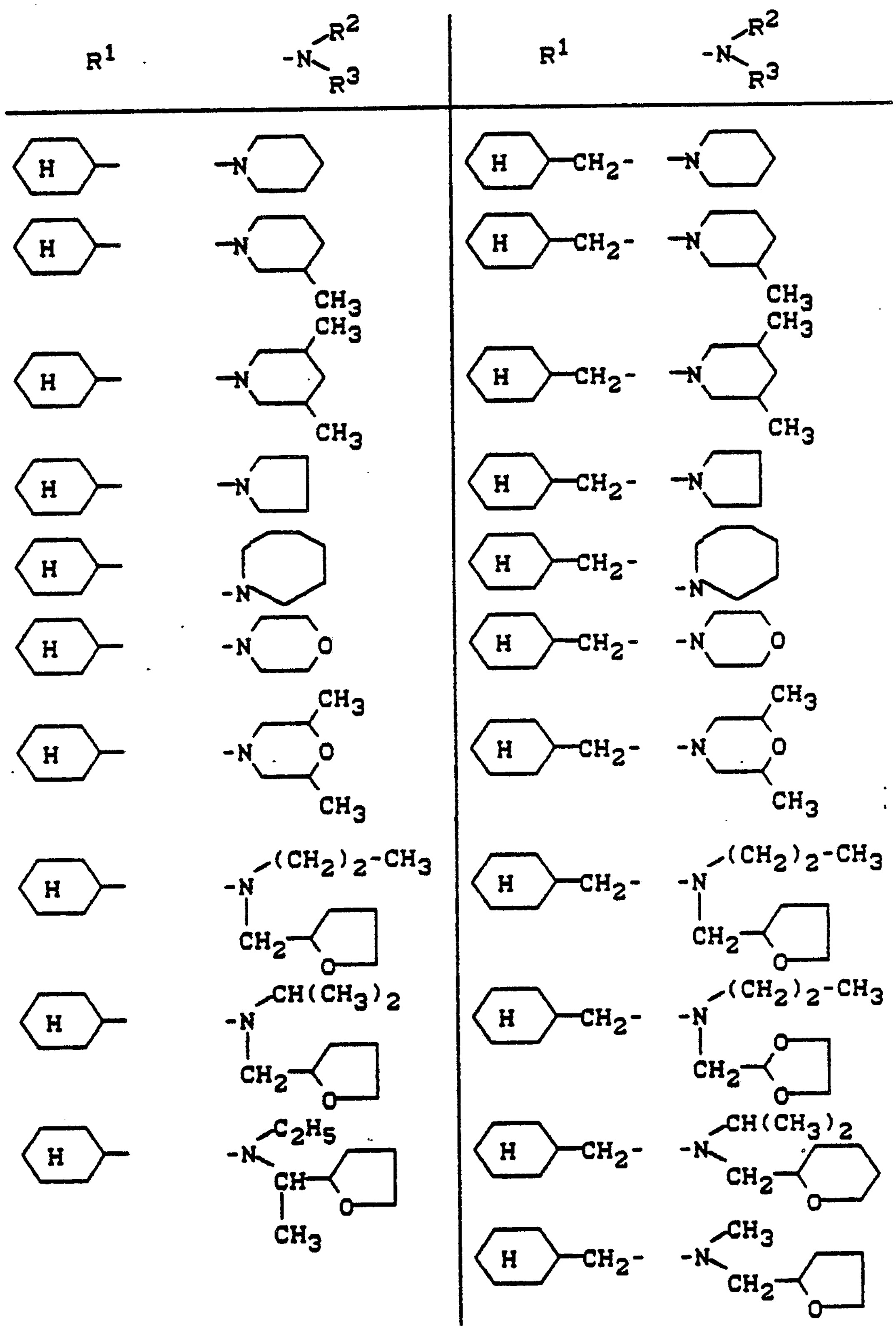

| $R^1$ | 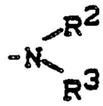 | $R^1$ | 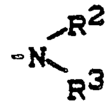 |
|---|---|---|---|
| 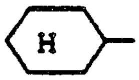 | 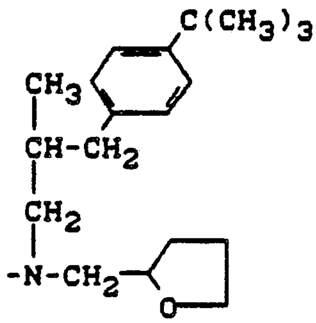 | 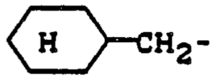 | 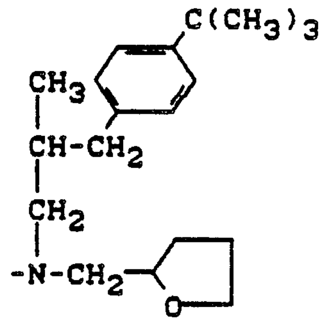 |
| 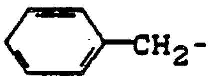 | 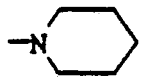 | 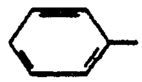 | 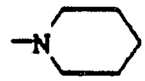 |
| 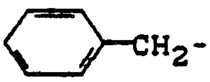 | 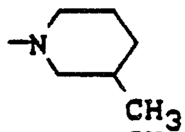 | 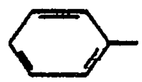 | 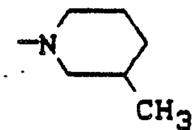 |
| 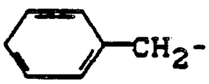 | 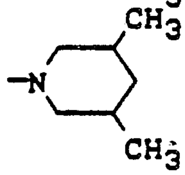 | 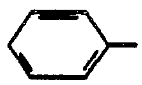 | 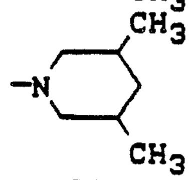 |
| 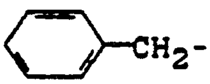 | 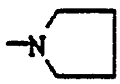 | 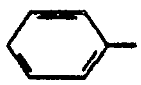 | 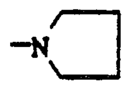 |
| 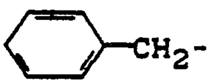 | 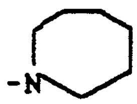 | 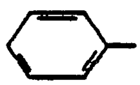 | 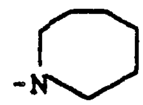 |
| 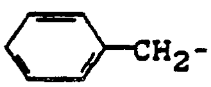 | 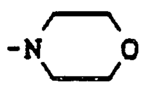 | 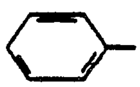 | 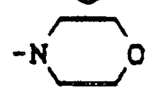 |
| 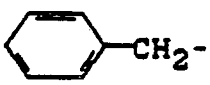 | 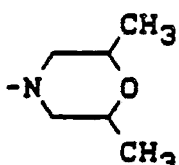 | 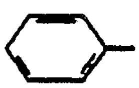 | 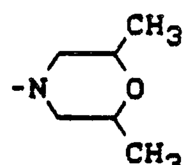 |
| 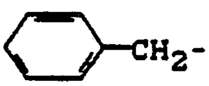 | 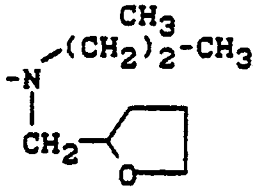 | 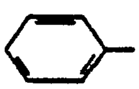 | 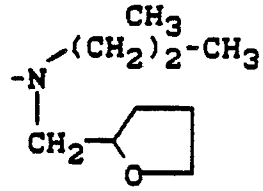 |

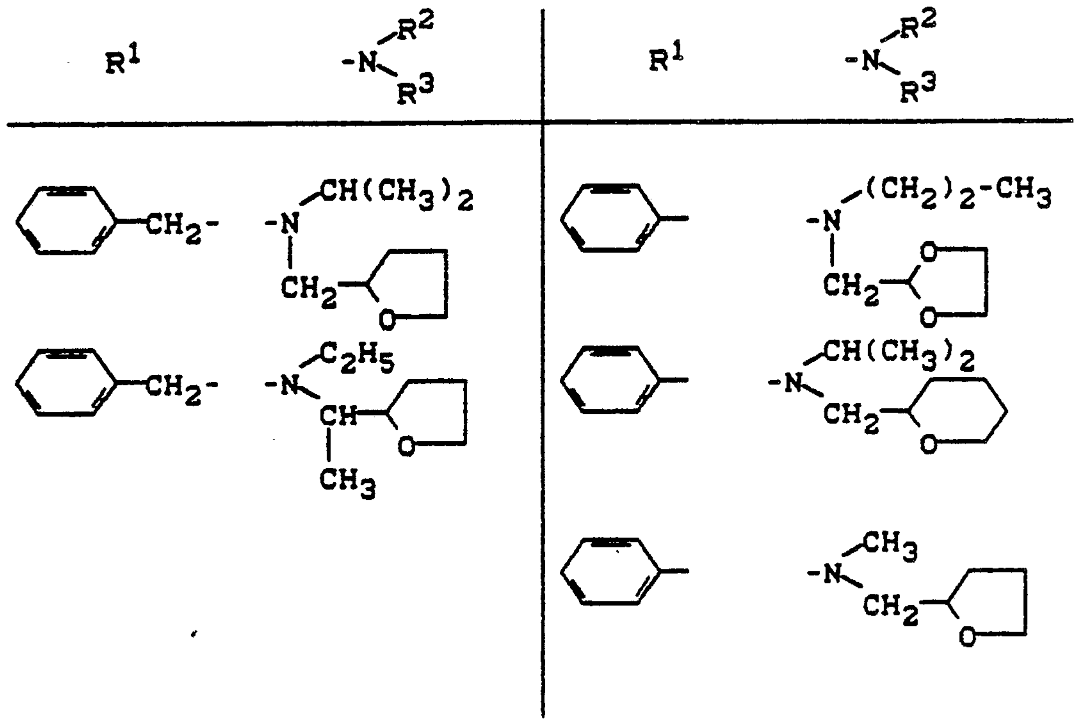

Verwendet man beispielsweise 4-t-Butylcyclohexanon und Piperidin als Ausgangsstoffe sowie Natriumcyanoborhydrid als Reduktionsmittel, so läßt sich der Reaktionsablauf des erfindungsgemäßen Verfahrens (a) durch das folgende Formelschema darstellen:

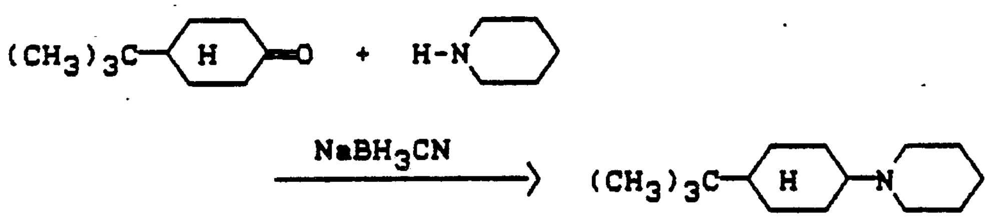


Verwendet man beispielsweise 4-(4-t-Butylphenyl)-2,6-dimethylmorpholin als Ausgangsverbindung und Ruthenium auf Aktivkohle als Katalysator, so läßt sich der Reaktionsablauf des erfindungsgemäßen Verfahrens (b) durch das folgende Formelschema darstellen:

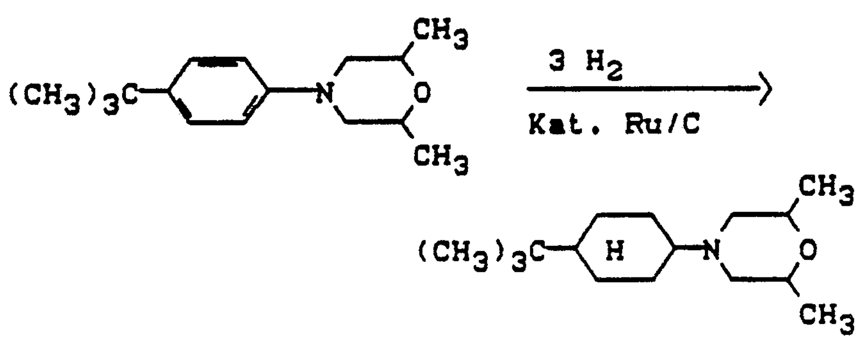


Verwendet man beispielsweise 4-Isopropylcyclohexylamin und Tetrahydrofuran-2-aldehyd als Ausgangsstoffe sowie molekularen Wasserstoff in Gegenwart von Palladium auf Aluminiumoxid als Reduktionsmittel, so läßt sich der Reaktionsablauf des erfindungsgemäßen Verfahrens (c) durch das folgende

Formelschema dastellen:

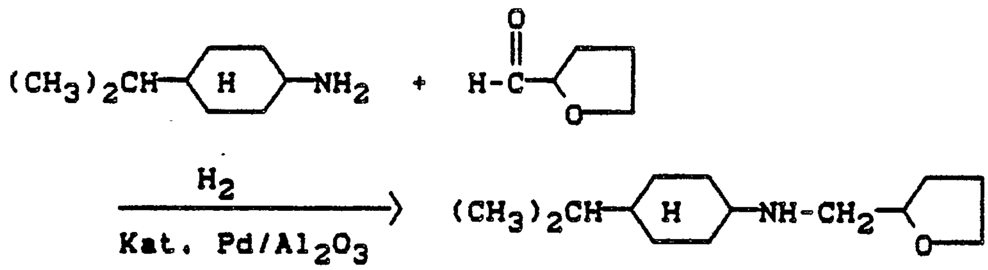


Verwendet man beispielsweise 4-t-Butyl-N-isopropylcyclohexylamin und 2-Tetrahydrofuranylmethylbromid als Ausgangsstoffe, so läßt sich der Reaktionsablauf des erfindungsgemäßen Verfahrens (d) durch das folgende Formelschema darstellen:

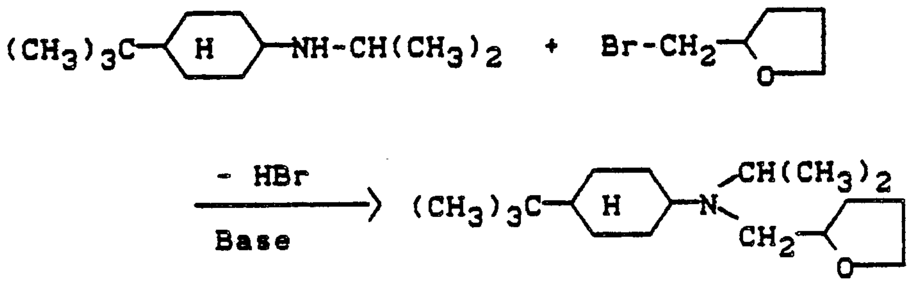


Verwendet man beispielsweise 4-t-Butyl-N-(2-tetrahydrofuranylmethyl)-cyclohexylamin und n-Propylbromid als Ausgangsstoffe, so läßt sich der Reaktionsablauf des erfindungsgemäßen Verfahrens (e) durch das folgende Formelschema darstellen:

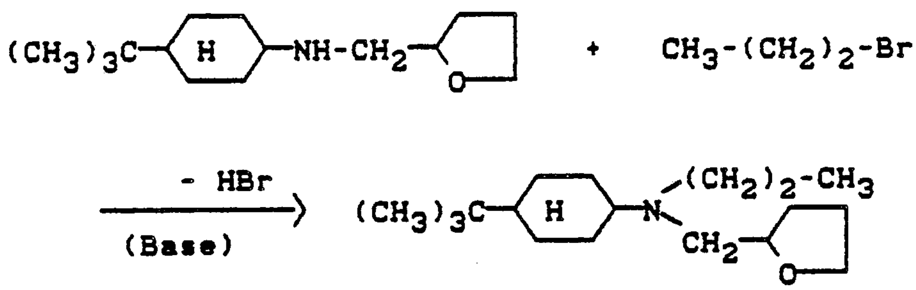


Die zur Durchführung des erfindungsgemäßen Verfahrens (a) als Ausgangsstoffe benötigten Cyclohexanone sind durch die Formel (II) allgemein definiert. In dieser Formel (II) steht $R^1$ vorzugsweise für diejenigen Reste, die bereits im Zusammenhang mit der Beschreibung der erfindungsgemäßen Stoffe der Formel (I) als bevorzugt für diese Substituenten genannt wurden.

Die Cyclohexanone der Formel (II) sind allgemein bekannte Verbindungen der organischen Chemie (vergl. z.B. Tetrahedron Lett. 1973, 919-922; J.org.Chem.33, 935-942 [1968]).

Die zur Durchführung des erfindungsgemäßen Verfahrens (a) weiterhin als Ausgangsstoffe benötigten Amine sind durch die Formel (III) allgemein definiert. In dieser Formel (III) stehen $R^2$ und $R^3$ vorzugsweise für diejenigen Reste, die bereits im Zusammenhang mit der Beschreibung der erfindungsgemäßen Stoffe der Formel (I) als bevorzugt für diese Substituenten genannt wurden.

Die Amine der Formel (III) sind ebenfalls allgemein bekannte Verbindungen der organischen Chemie (vergl. z.B. Rocz. Chem. 42, 353 [1968]; JP 60/146885).

Die zur Durchführung des erfindungsgemäßen Verfahrens (b) als Ausgangsstoffe benötigten substituierten Aniline sind durch die Formel (IV) allgemein definiert. In dieser Formel (IV) stehen $R^1$, $R^2$ und $R^3$ vorzugsweise für diejenigen Reste, die bereits im Zusammenhang mit der Beschreibung der erfindungsgemäßen Stoffe der Formel (I) als bevorzugt für diese Substituenten genannt wurden.

Die substituierten Aniline der Formel (IV) sind bekannt oder erhältlich in Analogie zu bekannten Verfahren (vergl. z.B. J. med. Chem. 22, 1460-1464 [1979]; US-PS 3 803 127).

Die zur Durchführung des erfindungsgemäßen Verfahrens (c) als Ausgangsstoffe benötigten Cyclohexylamine sind durch die Formel (V) allgemein definiert. In dieser Formel (V) steht $R^1$ vorzugsweise für diejenigen Reste, die bereits im Zusammenhang mit der Beschreibung der erfindungsgemäßen Stoffe der Formel (I) als bevorzugt für diese Substituenten genannt wurden.

Die Cyclohexylamine der Formel (V) sind bekannt oder erhältlich in Analogie zu bekannten Verfahren (vergl. z.B. J. org. Chem. 50, 5448-5450 [1985]).

Die zur Durchführung des erfindungsgemäßen Verfahrens (c) weiterhin als Ausgangsstoffe benötigten Aldehyde oder Ketone sind durch die Formel (VI) allgemein definiert. In dieser Formel (VI) steht

$R^4$ vorzugsweise für Wasserstoff oder für geradkettiges oder verzweigtes Alkyl mit 1 bis 4 Kohlenstoffatomen. $R^4$ steht insbesondere für Wasserstoff oder für Methyl.

$R^5$ steht vorzugsweise für Tetrahydrofuranyl, Tetrahydropyranyl, Dioxolanyl, Dioxanyl oder für Tetrahydrofuranylalkyl, Tetrahydropyranylalkyl, Dioxolanylalkyl oder Dioxanylalkyl mit jeweils 1 bis 4 Kohlenstoffatomen in den jeweiligen geradkettigen oder verzweigten Alkylteilen.

Die Aldehyde oder Ketone der Formel (VI) sind bekannt oder erhältlich in Analogie zu bekannten Verfahren (vergl. z.B. J.Heterocycl.Chem. 18, 565-569 [1981]; Tetrahedron 36, 1649-1665 [1980]; J.Amer.Chem.Soc. 95, 3635-3640 [1973]; Khim. Geterotsikl. Soedin 1968, 359 bzw. CA 69: 106362j; Agricult. Biol. Chem. 48, 2135-2136 [1984]; Helv. Chim. Acta 56, 3056-3059 [1973]).

Die zur Durchführung des erfindungsgemäßen Verfahrens (d) als Ausgangsstoffe benötigten Cyclohexylamine sind durch die Formel (VII) allgemein definiert. In dieser Formel (VII) steht $R^1$ vorzugsweise für diejenigen Reste, die bereits im Zusammenhang mit der Beschreibung der erfindungsgemäßen Stoffe der Formel (I) als bevorzugt für diese Substituenten genannt wurden.

$R^{3-1}$ steht vorzugsweise für Wasserstoff, für geradkettiges oder verzweigtes Alkyl mit 1 bis 6 Kohlenstoffatomen oder für unsubstituiertes oder einfach oder mehrfach, gleich oder verschieden substituiertes Aralkyl mit 1 bis 6 Kohlenstoffatomen im geradkettigen oder verzweigten Alkylteil und 6 bis 10 Kohlenstoffatomen im Arylteil, wobei als Arylsubstituenten infrage kommen: Halogen, Cyano, Nitro, jeweils geradkettiges oder verzweigtes Alkyl, Alkoxy oder Alkylthio mit jeweils 1 bis 4 Kohlenstoffatomen, jeweils geradkettiges oder verzweigtes Halogenalkyl, Halogenalkoxy oder Halogenalkylthio mit jeweils 1 bis 4 Kohlenstoffatomen und 1 bis 9 gleichen oder verschiedenen Halogenatomen, jeweils geradkettiges oder verzweigtes Alkoxycarbonyl oder Alkoximinoalkyl mit jeweils 1 bis 4 Kohlenstoffatomen in den einzelnen Alkylteilen sowie unsubstituiertes oder einfach oder mehrfach, gleich oder verschieden durch Halogen und/oder geradkettiges oder verzweigtes Alkyl mit 1 bis 4 Kohlenstoffatomen substituiertes Phenyl;

$R^{3-1}$ steht außerdem für Tetrahydrofuranylalkyl, Tetrahydropyranylalkyl, Dioxolanylalkyl oder Dioxanylalkyl mit jeweils 1 bis 4 Kohlenstoffatomen im geradkettigen oder verzweigten Alkylteil;

$R^{3-1}$ steht insbesondere für Wasserstoff, Methyl, Ethyl, n- oder i-Propyl, n-, i-, s- oder t-Butyl oder für unsubstituiertes oder ein- bis dreifach, gleich oder verschieden substituiertes Phenylalkyl mit 1 bis 4 Kohlenstoffatomen in geradkettigen oder verzweigten Alkylteil, wobei als Phenylsubstituenten infrage kommen: Fluor, Chlor, Brom, Cyano, Nitro, Methyl, Ethyl, n- oder i- Propyl, n-, i-, s- oder t-Butyl, Methoxy, Ethoxy, n-oder i-Propoxy, Methylthio, Trifluormethyl, Trifluormethoxy, Trifluormethylthio oder unsubstituiertes oder ein- bis dreifach, gleich oder verschieden durch Fluor, Chlor, Brom, Methyl und/oder Ethyl substituiertes Phenyl, außerdem für Tetrahydrofuranylmethyl, Tetrahydropyranylmethyl, Dioxolanylmethyl, Dioxanylmethyl.

Die Cyclohexylamine der Formel (VII) sind teilweise bekannt (vergl. z.B. J.org.Chem. 50, 5448-5450 [1985]; DE-OS 36 30 344; Synthesis 1987, 1005-1007; J.chem. Soc.C. 1967, 1509-1512), teilweise sind sie erfindungsgemäße Verbindungen und erhältlich mit Hilfe der erfindungsgemäßen Verfahren (a), (b) oder (c).

Die zur Durchführung des erfindungsgemäßen Verfahrens (d) weiterhin als Ausgangsstoffe benötigten Alkylierungsmittel sind durch die Formel (VIIIa) allgemein definiert. In dieser Formel (VIIIa) steht $R^{2-1}$ für Tetrahydrofuranylalkyl, Tetrahydropyranylalkyl, Dioxolanylalkyl oder Dioxanylalkyl mit jeweils 1 bis 4 Kohlenstoffatomen im geradkettigen oder verzweigten Alkylteil; insbesondere für Tetrahydrofuranylmethyl, Tetrahydropyranylmethyl, Dioxolanylmethyl oder Dioxanylmethyl. $E^1$ steht für einen bei Alkylierungsmitteln üblichen Abgangsrest wie beispielsweise Halogen, Alkylsulfonyloxy oder p-Toluolsulfonyloxy; $E^1$ steht insbesondere für Chlor, Brom oder Iod.

Die Alkylierungsmittel der Formel (VIIIa) sind bekannt oder erhältlich in Analogie zu bekannten Verbindungen (vergl. z.B. J.med.Chem. 1, 996-1000 [1975]; Bull. Soc. Chim. France 1971, 1080-1087; J.org.Chem. 34, 212-218 [1969]; Bull. Soc.chim. France 1972, 2445-2459; US 3 951 640; US 3 901 902).

Die zur Durchführung des erfindungsgemäßen Verfahrens (e) als Ausgangsstoffe benötigten substituierten Cyclohexylamine sind durch die Formel (Ic) allgemein definiert. In dieser Formel (Ic) steht $R^1$ vorzugsweise für diejenigen Reste, die bereits im Zusammenhang mit der Beschreibung der erfindungsgemäßen Stoffe der Formel (I) als bevorzugt für diese Substituenten genannt wurden.

$R^{2-1}$ steht vorzugsweise für Tetrahydrofuranylalkyl, Tetrahydropyranylalkyl, Dioxolanylalkyl oder Dioxanylalkyl mit jeweils 1 bis 4 Kohlenstoffatomen im geradkettigen oder verzweigten Alkylteil; insbesondere für Tetrahydrofuranylmethyl, Tetrahydropyranylmethyl, Dioxolanylmethyl oder Dioxanylmethyl.

Die substituierten Cyclohexylamine der Formel (Ic) sind erfindungsgemäße Verbindungen und erhältlich mit Hilfe der erfindungsgemäßen Verfahren (a), (b), (c) oder (d).

Die zur Durchführung des erfindungsgemäßen Verfahrens (e) weiterhin als Ausgangsstoffe benötigten Alkylierungsmittel sind durch die Formel (VIIIb) allgemein definiert. In dieser Formel (VIIIb) steht $R^{3-2}$ vorzugsweise für geradkettiges oder verzweigtes Alkyl mit 1 bis 6 Kohlenstoffatomen oder für gegebenenfalls einfach oder mehrfach, gleich oder verschieden substituiertes Aralkyl mit 1 bis 6 Kohlenstoffatomen im geradkettigen oder verzweigten Alkylteil und 6 bis 10 Kohlenstoffatomen im Arylteil, wobei als Arylsubstituenten infrage kommen: Halogen, Cyano, Nitro, jeweils geradkettiges oder verzweigtes Alkyl, Alkoxy oder Alkylthio mit jeweils 1 bis 4 Kohlenstoffatomen, jeweils geradkettiges oder verzweigtes Halogenalkyl, Halogenalkoxy oder Halogenalkylthio mit jeweils 1 bis 4 Kohlenstoffatomen und 1 bis 9 gleichen oder verschiedenen Halogenatomen, jeweils geradkettiges oder verzweigtes Alkoxycarbonyl oder Alkoximinoalkyl mit jeweils 1 bis 4 Kohlenstoffatomen in den einzelnen Alkylteilen sowie gegebenenfalls einfach oder mehrfach, gleich oder verschieden durch Halogen und/oder geradkettiges oder verzweigtes Alkyl mit 1 bis 4 Kohlenstoffatomen, substituiertes Phenyl; $R^{3-2}$ steht insbesondere für Wasser stoff, Methyl, Ethyl, n- oder i-Propyl, n-, i-, s- oder t-Butyl oder für gegebenenfalls ein- bis dreifach, gleich oder verschieden substituiertes Phenylalkyl mit 1 bis 4 Kohlenstoffatomen im geradkettigen oder verzweigten Alkylteil, wobei als Phenylsubstituenten infrage kommen: Fluor, Chlor, Brom, Cyano, Nitro, Methyl, Ethyl, n- oder i-Propyl, n-, i-, s- oder t-Butyl, Methoxy, Ethoxy, n- oder i-Propoxy, Methylthio, Trifluormethyl, Trifluormethoxy, Trifluormethylthio oder gegebenenfalls ein- bis dreifach, gleich oder verschieden durch Fluor, Chlor, Brom, Methyl und/oder Ethyl substituiertes Phenyl.

$E^2$ steht für einen bei Alkylierungsmitteln üblichen Abgangsrest, wie beispielsweise Halogen, Alkylsulfonyloxy, Alkoxysulfonyloxy oder p-Toluolsulfonyloxy; $E^2$ steht insbesondere für Chlor, Brom oder Iod.

Die Alkylierungsmittel der Formel (VIIIb) sind allgemein bekannte Verbindungen der organischen Chemie.

Zur Durchführung der erfindungsgemäßen Verfahren (a) und (c) ist die Gegenwart eines geeigneten Reduktionsmittels erforderlich. Als solche kommen alle üblicherweise für derartige reduktive Aminierungsreaktionen einsetzbaren Reduktionsmittel infrage.

Vorzugsweise verwendet man molekularen Wasserstoff in Gegenwart eines geeigneten Hydrierkatalysators, wie beispielsweise Raney-Nickel, Platin oder Palladium oder auch komplexe Hydride, wie beispielsweise Lithiumaluminiumhydrid, Natriumborhydrid, Natriumcyanoborhydrid oder komplexe Aluminiumhydride, wie Diisopropylaluminium hydrid oder Diisobutylaluminiumhydrid gegebenenfalls auch in Gegenwart von Natriumhydrid.

(Vergl. hierzu C.Ferri "Reaktionen der organischen Synthese" S.85f, 98f, 133; Thieme Verlag Stuttgart 1978 und dort zitierte Literatur sowie "Organikum" S.542f, VEB Deutscher Verlag der Wissenschaften, Berlin 1981).

Als Verdünnungsmittel zur Durchführung der erfindungsgemäßen Verfahren (a) und (c) kommen alle üblicherweise für derartige reduktive Aminierungsreaktionen einsetzbaren Verdünnungsmittel infrage. Mit besonderem Vorzug verwendet man halogenierte Kohlenwasserstoffe, wie Dichlormethan, Chloroform oder Tetrachlorkohlenstoff; Ether,wie Diethylether, Diisopropylether, Dioxan, Tetrahydrofuran oder Ethylenglykoldimethylether; Alkohole, wie Methanol, Ethanol, n- oder i-Propanol oder Ethylenglykolmonomethylether, gegebenenfalls auch in Mischung mit Wasser oder reines Wasser als Verdünnungsmittel.

Die erfindungsgemäßen Verfahren (a) und (c) können gegebenenfalls auch in einem Zweiphasensystem, wie beispielsweise Wasser/Chloroform oder Wasser/Dichlormethan, gegebenenfalls in Gegenwart eines Phasentransferkatalysators, durchgeführt werden. Als Beispiele für solche Katalysatoren seien genannt: Tetrabutylammoniumiodid, Tetrabutylammoniumbromid, Tributyl-methylphosphoniumbromid, Trimethyl-$C_{13}/C_{15}$-alkylammoniumchlorid, Dibenzyl-dimethyl-ammoniummethylsulfat, Dimethyl-$C_{12}/C_{14}$-alkyl-benzylammoniumchlorid, Tetrabutylammoniumhydroxid, 15-Krone-5, 18-Krone-6, Triethylbenzylammoniumchlorid, Trimethylbenzylammoniumchlorid, Tris-[2-(2-methoxyethoxy)-ethyl]-amin oder Methyltridecylammoniumchlorid.

Die erfindungsgemäßen Verfahren (a) und (c) können in Abhängigkeit vom verwendeten Reduktionsmittel gegebenenfalls auch in Gegenwart eines geeigneten Reaktionshilfsmittels durchgeführt werden. Als solche kommen alle üblichen anorganischen oder organischen Basen oder Säuren infrage. Hierzu gehören

als Basen beispielsweise Alkalimetallhydroxide, wie Natriumhydroxid oder Kaliumhydroxid, Alkalimetallcarbonate, wie Natriumcarbonat, Kaliumcarbonat oder Natriumhydrogencarbonat oder als Säuren anorganische Mineralsäure, wie Salzsäure oder Schwefelsäure oder organische Säuren, wie beispielsweise Essigsäure.

Die Reaktionstemperaturen können bei der Durchführung der erfindungsgemäßen Verfahren (a) und (c) in einem größeren Bereich variiert werden. Im allgemeinen arbeitet man bei Temperaturen zwischen -20 °C und 250 °C, vorzugsweise bei Temperaturen zwischen 0 °C und 150 °C.

Die erfindungsgemäßen Verfahren (a) und (c) können gegebenenfalls auch unter erhöhtem Druck durchgeführt werden. Vorzugsweise arbeitet man in diesen Fällen in Druckbereichen zwischen 1 und 300 bar, insbesondere zwischen 20 und 150 bar.

Zur Durchführung des erfindungsgemäßen Verfahrens (a) setzt man pro Mol an Cyclohexanon der Formel (II) im allgemeinen 1,0 bis 5,0 Mol, vorzugsweise 1,0 bis 3,0 Mol an Amin der Formel (III) und 1,0 bis 20,0 Mol, vorzugsweise 0,5 bis 5,0 Mol an Reduktionsmittel ein.

Die Reaktionsdurchführung, Aufarbeitung und Isolierung der Reaktionsprodukte erfolgt nach allgemein üblichen Methoden (vergl. auch die Herstellungsbeispiele).

Zur Durchführung des erfindungsgemäßen Verfahrens (c) setzt man pro Mol an Aldehyd oder Keton der Formel (VI) im allgemeinen 1,0 bis 5,0 Mol, vorzugsweise 1,0 bis 3,0 Mol an Cyclohexylamin der Formel (V) und gegebenenfalls 0,2 bis 20,0 Mol, vorzugsweise 0,5 bis 5,0 Mol an Reduktionsmittel ein.

Die Reaktionsdurchführung, Aufarbeitung und Isolierung der Reaktionsprodukte erfolgt nach allgemein üblichen Methoden.

Als Verdünnungsmittel zur Durchführung des erfindungsgemäßen Verfahrens (b) kommen alle unter Hydrierungsbedingungen inerten Solventien in Betracht. Vorzugsweise verwendbar sind Kohlenwasserstoffe, wie Petrolether, Pentan, Hexan, Heptan, Cyclohexan, Alkohole, wie Methanol, Ethanol, n-Propanol und i-Propanol oder Ether, wie Diethylether, Dioxan, Tetrahydrofuran oder Ethylenglykoldimethyl- oder -diethylether. Es kann jedoch auch ohne Verdünnungsmittel gearbeitet werden.

Als Hydrierungskatalysatoren können bei dem erfindungsgemäßen Verfahren (b) alle üblicherweise für derartige Reaktionen verwendbaren Katalysatoren eingesetzt werden. Vorzugsweise verwendet man Raney-Nickel oder Edelmetall katalysatoren, wie Palladium, Ruthenium, Palladiumoxid, Platin oder Platinoxid, gegebenenfalls auf einem geeigneten Trägerstoff, wie z.B. Kohle oder Aluminiumoxid.

Die Reaktionstemperaturen können bei dem erfindungsgemäßen Verfahren (b) in einem größeren Bereich variiert werden. Im allgemeinen arbeitet man bei Temperaturen zwischen 50 °C und 250 °C, vorzugsweise bei Temperaturen zwischen 80 °C und 200 °C.

Das erfindungsgemäße Verfahren (b) kann bei Normaldruck, aber auch bei erhöhtem Druck durchgeführt werden. Bevorzugt wird unter erhöhtem Druck gearbeitet. Im allgemeinen arbeitet man in Druckbereichen zwischen 5 und 300 atm, vorzugsweise zwischen 10 und 200 atm.

Bei der Durchführung des erfindungsgemäßen Verfahrens (b) setzt man pro Mol an substituiertem Anilin der Formel (IV) im allgemeinen 3,0 bis 30 Mol Wasserstoff und 0,001 bis 10 Mol, vorzugsweise 0,01 bis 0,1 Mol Katalysator ein.

Bei der Durchführung des erfindungsgemäßen Verfahrens (b) geht man im allgemeinen so vor, daß man die Verbindungen der Formel (IV) in Gegenwart eines Verdünnungsmittels bei der jeweils gewünschten Temperatur mit Wasserstoff in Gegenwart des Hydrierungskatalysators im Autoklaven umsetzt. Die Aufarbeitung und Isolierung der Reaktionsprodukte erfolgt nach üblichen Methoden.

Als Verdünnungsmittel zur Durchführung der erfindungsgemäßen Verfahren (d) und (e) kommen inerte organische Lösungsmittel oder wäßrige Systeme infrage. Hierzu gehören insbesondere aliphatische oder aromatische, gegebenenfalls halogenierte Kohlenwasserstoffe, wie beispielsweise Benzin, Benzol, Toluol, Xylol, Chlorbenzol, Petrolether, Hexan, Cyclohexan, Dichlormethan, Chloroform, Tetrachlorkohlenstoff, Ether, wie Diethylether, Dioxan, Tetrahydrofuran oder Ethylenglykoldimethyl- oder -diethylether, Ketone, wie Aceton oder Butanon, Nitrile, wie Acetonitril oder Propionitril, Amide, wie Dimethylformamid, Dimethylacetamid, N-Methylformanilid, N-Methylpyrrolidon oder Hexamethylphosphorsäuretriamid, Ester, wie Essigsäureethylester oder Sulfoxide, wie Dimethylsulfoxid.

Die erfindungsgemäßen Verfahren (d) und (e) können gegebenenfalls auch in einem Zweiphasensystem, wie beispielsweise Wasser/Toluol oder Wasser/Dichlormethan, gegebenenfalls in Gegenwart eines Phasentransferkatalysators durchgeführt werden. Als Beispiele für solche Katalysatoren seien genannt: Tetrabutylammoniumiodid, Tetrabutylammoniumbromid, Tributyl-methylphosphoniumbromid, Trimethyl-$C_{13}$/$C_{15}$-alkylammoniumchlorid, Dibenzyldimethyl-ammoniummethylsulfat, Dimethyl-$C_{12}$-$C_{14}$-alkyl-benzylammoniumchlorid, Tetrabutylammoniumhydroxid, 15-Krone-5, 18 Krone-6,Triethylbenzylammoniumchlorid, Trimethylbenzylammoniumchlorid. Es ist auch möglich, die erfindungsgemäßen Verfahren (d) und (e) ohne Zusatz eines Lösungsmittels durchzuführen.

Als Reaktionshilfsmittel zur Durchführung der erfin dungsgemäßen Verfahren (d) und (e) kommen alle

üblicherweise verwendbaren anorganischen und organischen Basen infrage. Vorzugsweise verwendet man Alkalimetallhydroxide, -carbonate oder -hydrogencarbonate, wie beispielsweise Natriumhydroxid, Natriumcarbonat oder Natriumhydrogencarbonat oder auch tertiäre Amine, wie beispielsweise Triethylamin, N,N-Dimethylanilin, Pyridin, 4-(N,N-Dimethylamino)-pyridin, Diazabicyclooctan (DABCO), Diazabicyclononen (DBN) oder Diazabicycloundecen (DBU).
Es ist auch möglich, die als Reaktionsteilnehmer verwendeten Cyclohexylamine der Formeln (VII) bzw. (Ic) in entsprechendem Überschuß gleichzeitig als Reaktionshilfsmittel einzusetzen.

Die Reaktionstemperaturen können bei der Durchführung der erfindungsgemäßen Verfahren (d) und (e) in einem größeren Bereich variiert werden. Im allgemeinen arbeitet man bei Temperaturen zwischen - 20 °C und + 180 °C, vorzugsweise bei Temperaturen zwischen 20 °C und +150 °C.

Zur Durchführung des erfindungsgemäßen Verfahrens (d) setzt man pro Mol an Cyclohexylamin der Formel (VII) im allgemeinen 1,0 bis 3,0 Mol, vorzugsweise 1,0 bis 1,5 Mol an Alkylierungsmittel der Formel (VIIIa) und gegebenenfalls 1,0 bis 3,0 Mol, vorzugsweise 1,0 bis 1,5 Mol an Reaktionshilfsmittel, sowie gegebenenfalls 0,1 bis 1,0 Mol an Phasentransferkatalysator ein.

Zur Durchführung des erfindungsgemäßen Verfahrens (e) setzt man pro Mol an substituiertem Cyclohexylamin der Formel (Ia) im allgemeinen 1,0 bis 5,0 Mol, vorzugsweise 1,0, bis 2,0 Mol an Alkylierungsmittel der Formel (VIIIb) und 1,0 bis 5,0 Mol, vorzugsweise 1,0 bis 2,0 Mol an Reaktionshilfsmittel sowie gegebenenfalls 0,1 bis 1,0 Mol an Phasentransferkatalysator ein.
Die Reaktionsdurchführung, Aufarbeitung und Isolierung der Reaktionsprodukte der Formel (Ib) erfolgt in beiden Fällen nach üblichen Methoden.

Die Säureadditionssalze der Verbindungen der Formel (I) können in einfacher Weise nach üblichen Salzbildungsmethoden, wie z.B. durch Lösen einer Verbindung der Formel (I) in einem geeigneten inerten Lösungsmittel und Hinzufügen der Säure, wie z.B. Chlorwasserstoffsäure, erhalten werden und in bekannter Weise, z.B. durch Abfiltrieren, isoliert und gegebenenfalls durch Waschen mit einem inerten organischen Lösungsmittel gereinigt werden.

Die erfindungsgemäßen Wirkstoffe weisen eine starke Wirkung gegen Schädlinge auf und können zur Bekämpfung von unerwünschten Schadorganismen praktisch eingesetzt werden. Die Wirkstoffe sind für den Gebrauch als Pflanzenschutzmittel, insbesondere als Fungizide, geeignet.
Fungizide Mittel im Pflanzenschutz werden eingesetzt zur Bekämpfung von Plasmodiophoromycetes, Oomycetes, Chytridiomycetes, Zygomycetes, Ascomycetes, Basidiomycetes, Deuteromycetes.

Beispielhaft aber nicht begrenzend seien einige Erreger von pilzlichen Erkrankungen, die unter die oben aufgezählten Oberbegriffe fallen, genannt:
Pythium-Arten, wie beispielsweise Pythium ultimum;
Phytophthora-Arten, wie beispielsweise Phytophthora infestans;
Pseudoperonospora-Arten, wie beispielsweise Pseudoperonospora humuli oder Pseudoperonospora cubensis;
Plasmopara-Arten, wie beispielsweise Plasmopara viticola;
Peronospora-Arten, wie beispielsweise Peronospora pisi oder P. brassicae;
Erysiphe-Arten, wie beispielsweise Erysiphe graminis; Sphaerotheca-Arten, wie beispielsweise Sphaerotheca fuliginea;
Podosphaera-Arten, wie beispielsweise Podosphaera leucotricha;
Venturia-Arten, wie beispielsweise Venturia inaequalis; Pyrenophora-Arten, wie beispielsweise Pyrenophora teres oder P. graminea
(Konidienform: Drechslera, Syn: Helminthosporium);
Cochliobolus-Arten, wie beispielsweise Cochliobolus sativus
(Konidienform: Drechslera, Syn: Helminthosporium);
Uromyces-Arten, wie beispielsweise Uromyces appendiculatus;
Puccinia-Arten, wie beispielsweise Puccinia recondita;
Tilletia-Arten, wie beispielsweise Tilletia caries;
Ustilago-Arten, wie beispielsweise Ustilago nuda oder Ustilago avenae;
Pellicularia-Arten, wie beispielsweise Pellicularia sasakii;
Pyricularia-Arten, wie beispielsweise Pyricularia oryzae;
Fusarium-Arten, wie beispielsweise Fusarium culmorum;
Botrytis-Arten, wie beispielsweise Botrytis cinerea;
Septoria-Arten, wie beispielsweise Septoria nodorum;
Leptosphaeria-Arten, wie beispielsweise Leptosphaeria nodorum;
Cercospora-Arten, wie beispielsweise Cercospora canescens;
Alternaria-Arten, wie beispielsweise Alternaria brassicae;

Pseudocercosporella-Arten, wie beispielsweise Pseudocercosporella herpotrichoides.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut, und des Bodens.

Die erfindungsgemäßen Wirkstoffe können mit besonders gutem Erfolg zur Bekämpfung von Getreidekrankheiten, wie beispielsweise gegen den Erreger des echten Getreidemehltaus (Erysiphe graminis) eingesetzt werden.

Die Wirkstoffe können in Abhängigkeit von ihren jeweiligen physikalischen und/oder chemischen Eigenschaften in übliche Formulierungen übergeführt werden, wie Lösungen, Emulsionen, Suspensionen, Pulver, Schäume, Pasten, Granulate, Aerosole, Feinstverkapselungen in polymeren Stoffen und in Hüllmassen für Saatgut, sowie ULV-Kalt-und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid; als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylarylpolyglykol-Ether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können in den Formulierungen im Mischung mit anderen bekannten Wirkstoffen vorliegen wie Fungizide, Insektizide, Akarizide und Herbizide sowie in Mischungen mit Düngemitteln und Wachstumsregulatoren.

Die Wirkstoffe konnen als solche, in Form ihrer Formulierungen oder den daraus bereiteten Anwendungsformen wie gebrauchsfertige Lösungen, Suspensionen, Spritzpulver, Pasten, lösliche Pulver, Stäubemittel und Granulate angewendet werden. Die Anwendung geschieht in üblicher Weise, z.B. durch Gießen, Verspritzen, Versprühen, Verstreuen, Verstäuben, Verschäumen, Bestreichen usw.. Es ist ferner möglich, die Wirkstoffe nach dem Ultra-Low-Volume-Verfahren auszubringen oder die Wirkstoffzubereitung oder den Wirkstoff selbst in den Boden zu injizieren. Es kann auch das Saatgut der Pflanzen behandelt werden.

Bei der Behandlung von Pflanzenteilen können die Wirkstoffkonzentrationen in den Anwendungsformen in einem größeren Bereich variiert werden. Sie liegen im allgemeinen zwischen 1 und 0,0001 Gew.-%, vorzugsweise zwischen 0,5 und 0,001 %.

Bei der Saatgutbehandlung werden im allgemeinen Wirkstoffmengen von 0,001 bis 50 g je Kilogramm Saatgut, vorzugsweise 0,01 bis 10 g benötigt.

Bei Behandlung des Bodens sind Wirkstoffkonzentrationen von 0,00001 bis 0,1 Gew.-%, vorzugsweise

von 0,0001 bis 0,02 % am Wirkungsort erforderlich.

Herstellungsbeispiele:

Beispiel1:

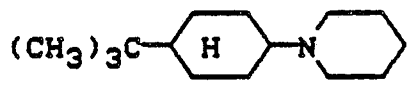

(Verfahren a)

Eine Mischung aus 15,4 g (0,1 Mol) 4-t-Butylcyclohexanon und 15 g (0,17 Mol) Piperidin in 75 ml Methanol wird mit 3 g Palladium auf Aluminiumoxid (1 prozentig) versetzt und bei 100 °C und 50 bar 3 Stunden mit Wasserstoff hydriert. Zur Aufarbeitung wird der Katalysator abfiltriert und das Filtrat im Vakuum eingeengt.

Man erhält 20,4 g (92 % der Theorie) an 1-(4-t-Butylcyclohexyl)-piperidin als Öl vom Brechungsindex $n_D^{20}$ 1,4802 in Form eines cis-trans-Gemisches, welches chromatographisch getrennt werden kann.

Beispiel 2:

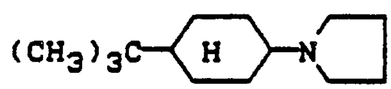

(Verfahren a)

Zu 42,6 g (0,6 Mol) Pyrrolidin in 200 ml Dichlormethan gibt man nacheinander 80 ml (0,2 Mol) 2,5 N Salzsäure in Methanol, 15,4 g (0,1 Mol) 4-6-Butylcyclohexanon, 29,3 g (0,07 Mol) Methyltridecylammoniumchlorid, 10 g 4Å-Molekularsieb und dann portionsweise unter Rühren 4,4 g (0,07 Mol) Natriumcyanoborhydrid, rührt nach beendeter Zugabe 2 Tage bei Raumtemperatur, stellt dann den pH-Wert mit Salzsäure auf 3 bis 4 ein, extrahiert zweimal mit Ether, stellt die wässrige Phase anschließend mit Natronlauge alkalisch, extrahiert erneut zweimal mit Ether, trocknet die alkalischen Etherextrakte über Natriumsulfat, engt im Vakuum ein und chromatographiert den Rückstand an Kieselgel (Laufmittel: Petrolether/ Essigester 3:1).

Man erhält 5,5 g eines Isomeren (1) vom Schmelzpunkt 50 °C, 6,0 g eines Isomeren (2) vom Schmelzpunkt 72 °C und 2,5 g einer Mischfraktion aus beiden Isomeren, so daß eine Gesamtausbeute von 14 g (72 % der Theorie) an 1-(4-t-Butylcyclohexyl)-pyrrolidin resultiert.

In entsprechender Weise und gemäß den allgemeinen Angaben zur Herstellung erhält man die folgenden substituierten Cyclohexylamine der allgemeinen Formel (I):

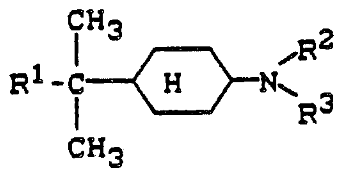 (I)

| Bsp. Nr. | 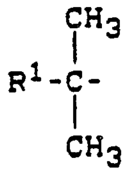 | 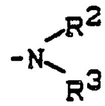 | Brechungs-index |
|---|---|---|---|
| 3 | $(CH_3)_3C$- | 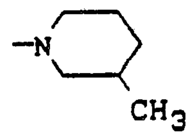 | $n_D^{20}$ : 1,4746 |
| 4 | $(CH_3)_3C$- | 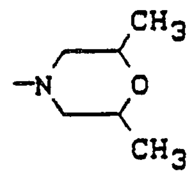 | $n_D^{20}$ : 1,4728 (Isomeres 1) |
| 5 | $(CH_3)_3C$- | 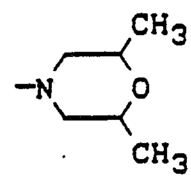 | $n_D^{20}$ : 1,4732 (Isomeres 2) |
| 6 | $(CH_3)_3C$- | 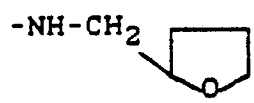 | $n_D^{25}$ : 1,4741 |
| 7 | $(CH_3)_3C$- | 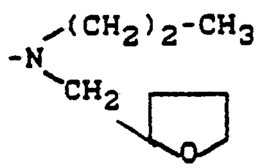 | $n_D^{25}$ : 1,4759 (Isomeres 1) |
| 8 | $(CH_3)_3C$- | 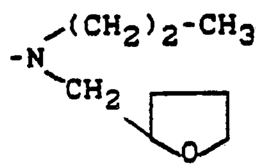 | $n_D^{25}$ : 1,4738 (Isomeres 2) |

Anwendungsbeispiele:

In dem folgenden Anwendungsbeispiel wurde die nachstehend aufgeführte Verbindung als Vergleichssubstanz eingesetzt:

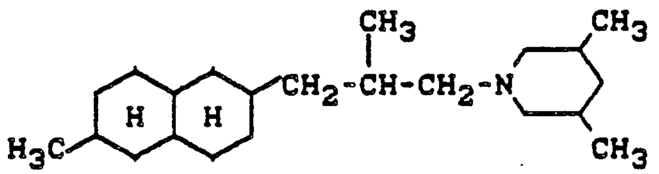

(A)

**2-[2-Methyl-3-(3,5-dimethylpiperidin-1-yl)-propyl]-6-methyldecalin**
**(bekannt aus EP 254 150)**

Beispiel A

Erysiphe-Test (Weizen) / protektiv

Lösungsmittel: 100 Gewichtsteile Dimethylformamid
Emulgator: 0,25 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit besprüht man junge Pflanzen mit der Wirkstoffzubereitung taufeucht. Nach Antrocknen des Spritzbelages werden die Pflanzen mit Sporen von Erysiphe graminis f.sp.tritici bestäubt.

Die Pflanzen werden in einem Gewächshaus bei einer Temperatur von ca. 20 °C und einer relativen Luftfeuchtigkeit von ca. 80 % aufgestellt, um die Entwicklung von Mehltauspusteln zu begünstigen.

7 Tage nach der Inokulation erfolgt die Auswertung.

Gegenüber der Verbindung (A) zeigen die Verbindungen der Herstellungsbeispiele (1), (2) und (5) bei einer beispielhaften Konzentration von 0,0025 Gew.-% einen um 40 bis 50 % höheren Wirkungsgrad.

**Ansprüche**

1. Substituierte Cyclohexylamine der allgemeinen Formel (I),

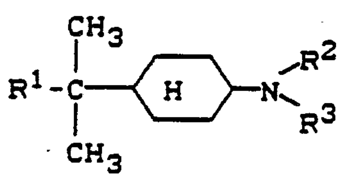

(I)

in welcher
$R^1$ für Wasserstoff, Alkyl, Cycloalkyl, Cycloalkylalkyl, Aralkyl oder Aryl steht,
$R^2$ für Tetrahydrofuranylalkyl, Tetrahydropyranylalkyl, Dioxolanylalkyl oder Dioxanylalkyl steht und
$R^3$ für Wasserstoff, Alkyl, für unsubstituiertes oder substituiertes Aralkyl, für Tetrahydrofuranylalkyl, Tetrahydropyranylalkyl, Dioxolanylalkyl oder Dioxanylalkyl steht oder
$R^2$ und $R^3$ gemeinsam mit dem Stickstoffatom, an welches sie gebunden sind, für einen unsubstituierten oder substituierten Heterocyclus stehen,
sowie deren Säureadditionssalze.

2. Cyclohexylamine gemäß Anspruch 1, wobei in der Formel (I)
$R^1$ für Wasserstoff, für geradkettiges oder verzweigtes Alkyl mit 1 bis 6 Kohlenstoffatomen, für Cycloalkyl

mit 3 bis 7 Kohlenstoffatomen, für Cycloalkylalkyl mit 3 bis 7 Kohlenstoffatomen im Cycloalkylteil und 1 bis 6 Kohlenstoffatomen im geradkettigen oder verzweigten Alkylteil, für Phenylalkyl mit 1 bis 6 Kohlenstoffatomen im geradkettigen oder verzweigten Alkylteil oder für Phenyl oder Naphthyl steht,

$R^2$ für Tetrahydrofuranylalkyl, Tetrahydropyranylalkyl, Dioxolanylalkyl oder Dioxanylalkyl mit jeweils 1 bis 4 Kohlenstoffatomen im geradkettigen oder verzweigten Alkylteil steht und

$R^3$ für Wasserstoff, für geradkettiges oder verzweigtes Alkyl mit 1 bis 6 Kohlenstoffatomen oder für unsubstituiertes oder einfach oder mehrfach, gleich oder verschieden substituiertes Aralkyl mit 1 bis 6 Kohlenstoffatomen im geradkettigen oder verzweigten Alkylteil und 6 bis 10 Kohlenstoffatomen im Arylteil steht, wobei als Arylsubstituenten infrage kommen:

Halogen, Cyano, Nitro, jeweils geradkettiges oder verzweigtes Alkyl, Alkoxy oder Alkylthio mit jeweils 1 bis 4 Kohlenstoffatomen, jeweils geradkettiges oder verzweigtes Halogenalkyl, Halogenalkoxy oder Halogenalkylthio mit jeweils 1 bis 4 Kohlenstoffatomen und 1 bis 9 gleichen oder verschiedenen Halogenatomen, jeweils geradkettiges oder verzweigtes Alkoxycarbonyl oder Alkoximinoalkyl mit jeweils 1 bis 4 Kohlenstoffatomen in den einzelnen Alkylteilen sowie unsubstituiertes oder einfach oder mehrfach, gleich oder verschieden durch Halogen und/oder geradkettiges oder verzweigtes Alkyl mit 1 bis 4 Kohlenstoffatomen substituiertes Phenyl; oder für Tetrahydrofuranylalkyl, Tetrahydropyranylalkyl, Dioxolanylalkyl oder Dioxanylalkyl mit jeweils 1 bis 4 Kohlenstoffatomen im geradkettigen oder verzweigten Alkylteil steht oder

$R^2$ und $R^3$ gemeinsam mit dem Stickstoffatom, an welches sie gebunden sind, für einen unsubstituierten oder einfach oder mehrfach, gleich oder verschieden substituierten gesättigten fünf- bis seibengliedrigen Heterocyclus stehen, der gegebenenfalls ein weiteres Heteroatom enthalten kann, wobei als Substituenten infrage kommen: jeweils geradkettiges oder verzweigtes Alkyl oder Hydroxyalkyl mit jeweils 1 bis 4 Kohlenstoffatomen.

3. Cyclohexylamine gemäß Anspruch 1, wobei in der Formel (I)

$R^1$ für Wasserstoff, Methyl, Ethyl, n- oder i-Propyl, Cyclopentyl, Cyclohexyl, Cyclohexylmethyl, Cyclohexylethyl, Cyclohexylpropyl, Phenyl, Benzyl, Phenylethyl oder Phenylpropyl steht,

$R^2$ für Tetrahydrofuranylmethyl, Tetrahydropyranylmethyl, Dioxolanylmethyl oder Dioxanylmethyl steht und

$R^3$ für Wasserstoff, Methyl, Ethyl, n- oder i-Propyl, n-, i-, s- oder t-Butyl oder für unsubstituiertes oder ein- bis dreifach, gleich oder verschieden substituiertes Phenylalkyl mit 1 bis 4 Kohlenstoffatomen im geradkettigen oder verzweigten Alkylteil steht, wobei als Phenylsubstituenten infrage kommen:

Fluor, Chlor, Brom, Cyano, Nitro, Methyl, Ethyl, n- oder i-Propyl, n-, i-, s- oder t-Butyl, Methoxy, Ethoxy, n- oder i-Propoxy, Methylthio, Trifluormethyl, Trifluormethoxy, Trifluormethylthio oder unsubstituiertes oder ein bis dreifach, gleich oder verschieden durch Fluor, Chlor, Brom, Methyl und/oder Ethyl substituiertes Phenyl; oder für Tetrahydrofuranylmethyl, Tetrahydropyranylmethyl, Dioxolanylmethyl, Dioxanylmethyl steht oder

$R^2$ und $R^3$ gemeinsam mit dem Stickstoffatom, an welches sie gebunden sind, für einen unsubstituierten oder ein- bis dreifach, gleich oder verschieden durch Methyl, Ethyl und/oder Hydroxymethyl substituierten 1-Pyrrolidinylrest, 1-Piperidinylrest, 1-Perhydroazepinylrest oder 4-Morpholinylrest stehen.

4. Verfahren zur Herstellung von substituierten Cyclohexylaminen der allgemeinen Formel (I),

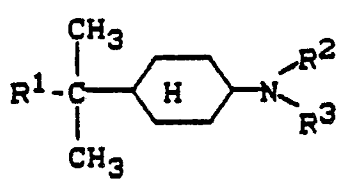

(I)

in welcher

$R^1$ für Wasserstoff, Alkyl, Cycloalkyl, Cycloalkylalkyl, Aralkyl oder Aryl steht,

$R^2$ für Tetrahydrofuranylalkyl, Tetrahydropyranylalkyl, Dioxolanylalkyl oder Dioxanylalkyl steht und

$R^3$ für Wasserstoff, Alkyl, für unsubstituiertes oder substituiertes Aralkyl, für Tetrahydrofuranylalkyl, Tetrahydropyranylalkyl, Dioxolanylalkyl oder Dioxanylalkyl steht oder

$R^2$ und $R^3$ gemeinsam mit dem Stickstoffatom, an welches sie gebunden sind, für einen unsubstituierten oder substituierten Heterocyclus stehen,

sowie deren Säureadditionssalze, dadurch gekennzeichnet, daß man

(a) Cyclohexanone der Formel (II),

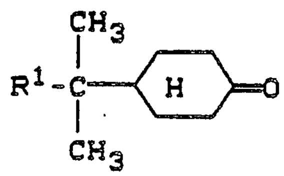

in welcher
$R^1$ die oben angegebene Bedeutung hat,
mit Aminen der Formel (III),

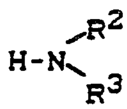

(III)

in welcher
$R^2$ und $R^3$ die oben angegebene Bedeutung haben,
in Gegenwart eines Reduktionsmittels, gegebenenfalls in Gegenwart eines Verdünnungsmittels und gegebenenfalls in Gegenwart eines Reaktionshilfsmittels umsetzt, oder daß man
(b) substituierte Aniline der Formel (IV),

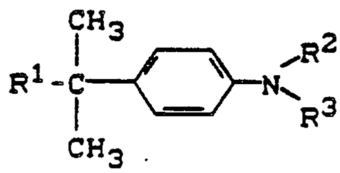

(IV)

in welcher
$R^1$, $R^2$ und $R^3$ die oben angegebene Bedeutung haben,
mit Wasserstoff in Gegenwart eines Katalysators und gegebenenfalls in Gegenwart eines Verdünnungsmittels hydriert;
und gegebenenfalls anschließend eines Säure addiert.

5. Verfahren zur Herstellung von substituierten Cyclohexylaminen der Formel (Ia),

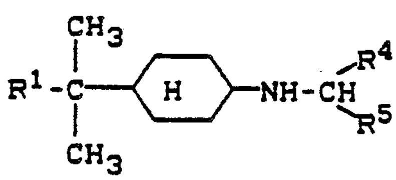

(Ia)

in welcher
$R^4$ für Wasserstoff oder Alkyl steht,
$R^5$ für Tetrahydrofuranyl, Tetrahydropyranyl, Dioxolanyl, Dioxanyl, Tetrahydrofuranylalkyl, Tetrahydropyranylalkyl, Dioxolanylalkyl oder Dioxanylalkyl steht und
$R^1$ die in Anspruch 4 angegebene Bedeutung hat,
dadurch gekennzeichnet, daß man Cyclohexylamine der Formel (V),

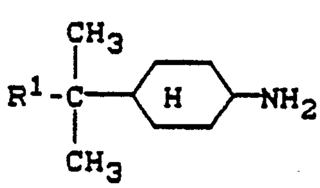

(V)

in welcher
$R^1$ die oben angegebene Bedeutung hat,
mit Aldehyden oder Ketonen der Formel (VI),

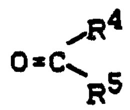

(VI)

in welcher
$R^4$ und $R^5$ die oben angegebene Bedeutung haben,
in Gegenwart eines Reduktionsmittels, gegebenenfalls in Gegenwart eines Verdünnungsmittels und gegebenenfalls in Gegenwart eines Reaktionshilfsmittels umsetzt, und gegebenenfalls anschließend eine Säure addiert.

6. Verfahren zur Herstellung von substituerten Cyclohexylaminen der Formel (Ib),

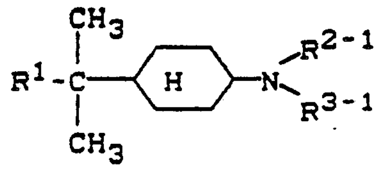

(Ib)

in welcher
$R^{2-1}$ für Tetrahydrofuranylalkyl, Tetrahydropyranylalkyl, Dioxolanylalkyl oder Dioxanylalkyl steht,
$R^{3-1}$ für Wasserstoff, Alkyl, für unsubstituiertes oder substituiertes Aralkyl, für Tetrahydrofuranylalkyl, Tetrahydropyranylalkyl, Dioxolanylalkyl oder Dioxanylalkyl steht und
$R^1$ die in Anspruch 4 angegebene Bedeutung hat,
dadurch gekennzeichnet, daß man
Cyclohexylamine der Formel (VII) oder (Ic),

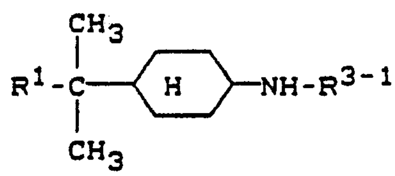

(VII)

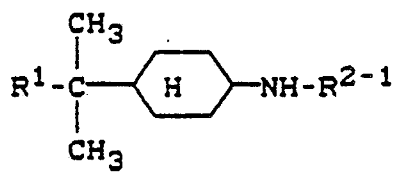

(Ic)

in welcher
$R^1$, $R^{2-1}$ und $R^{3-1}$ die oben angegebene Bedeutung haben,
mit Alkylierungsmitteln der Formel (VIIIa) oder (VIIIb)
$R^{2-1}$ - $E^1$ (VIIIa)
$R^{3-1}$ - $E^2$ (VIIIb)
in welcher
$R^{2-1}$ die oben angegebene Bedeutung hat und
$R^{3-2}$ für Alkyl oder für unsubstituiertes oder substituiertes Aralkyl steht und
$E^1$ und $E^2$ für eine elektronenanziehende Abgangsgruppe stehen,
jeweils gegebenenfalls in Gegenwart eines Verdünnungsmittels und gegebenenfalls in Gegenwart eines Reaktionshilfsmittels umsetzt und gegebenenfalls anschließbend eine Säure addiert.

7. Schädlingsbekämpfungsmittel, gekennzeichnet durch einen Gehalt an mindestens einem Cyclohexylamin der Formel (I) nach den Ansprüchen 1 bis 6.

8. Verwendung von Cyclohexylaminen der Formel (I) nach den Ansprüchen 1 bis 6 zur Bekämpfung von Schädlingen.

9. Verfahren zur Bekämpfung von Schädlingen, dadurch gekennzeichnet, daß man Cyclohexylamine der Formel (I) nach den Ansprüchen 1 bis 6 auf Schädlinge und/oder ihren Lebensraum einwirken läßt.

10. Verfahren zur Herstellung von Schädlingsbekämpfungsmitteln, dadurch gekennzeichnet, daß man Cyclohexylamine der Formel (I) nach den Ansprüchen 1 bis 6 mit Streckmitteln und/oder oberflächenaktiven Mitteln vermischt.